# EUROPEAN PATENT APPLICATION

(11) **EP 1 178 104 A1**
(43) Date of publication of application: **06.02.2002**
(21) Application number: 00116729.5
(22) Date of filing: 03.08.2000
(51) Int. Cl.: C11B 9/00, C11B 9/02, A23L 1/222

(54) **Use of essential oils for combatting GI tract infection by helicobacter-like organisms**

(71) Applicant: Société des Produits Nestlé S.A., 1800 Vevey (CH)
(72) Inventor: The designation of the inventor has not yet been filed
(74) Representative: Becker Kurig Straus

(57) **Abstract**

A nutritional composition comprising a specific essential oil and/or specific pure compound isolated from the essential oil for prevention or treatment of infection by an Helicobacter-like organism, a method of production of the composition, use of the composition in the manufacture of a functional food or medicament for the prevention or treatment of infection by an Helicobacter-like organism and a method of treatment of infection by an Helicobacter-like organism which comprises consumption or administration of a functional food or medicament comprising an effective amount of the composition.

## Description

The present invention relates to a nutritional composition comprising an essential oil for prevention or treatment of infection by an Helicobacter-like organism, a method of production of the composition, use of the composition in the manufacture of a functional food or medicament for the prevention or treatment of infection by an Helicobacter-like organism and a method of treatment of infection by an Helicobacter-like organism which comprises consumption or administration of a functional food or medicament comprising an effective amount of the composition.

Within the context of this specification the word "comprises" is taken to mean "includes, among other things". It is not intended to be construed as "consists of only".

Infection by an Helicobacter-like organism (GHLO) is thought to affect about 50% of the worlds population. A number of factors could be involved which determine whether an individual is subject to infection. These include genetic factors, environment, dietary habits, acquisition age and the strains of bacteria *(H. pylori)* involved. 35% of cases of infection can lead to chronic superficial gastritis; 10-15% can lead to peptic ulcers; 0.1% to 1% can lead to atrophic gastritis which can in turn lead to gastric cancer; and the remaining cases can lead to malt lymphoma. In addition, it has been suggested that *H. pylori* is involved in development of other conditions in adults including cardiovascular diseases (ischaemia / atherogenesis), autoimmune diseases (rheumatoid arthritis / thyroid immune disease), chronic urticaria, liver diseases (cirrhosis and hepatic encephalopathy), and gall-bladder cholelitiasis (Cag+ strains). Furthermore, in children it has been suggested that *H. pylori* is involved in development of other conditions including food allergy and iron-deficiency anemia (rare).

The prevalence of infection by GHLOs is high: in developed countries 5-15% of children and 20-65% of adults are estimated to be infected. In developing countries the figures are substantially higher with 13-70% in the 0-20 year old age group and 70-94% in the over 30 year old age group. In total it is estimated that more than 2.5 billion people are infected.

Companion animals also are infected by GHLOs.The most prevalent GHLOs in dogs are *H. bizzozeronii* and *H. salomonis*. For cats the most prevalent GHLOs are *H. heilmannii* and *H. felis*. In addition, cats are known to be able to transmit *H. heilmannii* to humans.

Therefore, it is clear that a need exists for an effective composition for combating GHLO infection.

The present invention addresses the problems set out above.

Remarkably, it has now been found that specific essential oils have an effect on GHLOs. In particular it has suprisingly been found that specific essential oils have a extraordinary strong effect. In addition, it has suprisingly been found that a number of essential oils have a remarkably strong effect on specific GHLOs.

An essential oil (EO) is an odorous product of a plant secondary metabolism. Generally it can be isolated by steam distillation from plants (leaves, stems, flowers, barks or roots) to result in an oily liquid at room temperatures.
Typically, the distilled liquid is a complex mix of volatile compounds (VC) such as phenols, alcohols, aldehydes, terpenes etc. The activity of the EO can vary with composition of the VCs present and this can vary with regard to the origin of the plant depending on geography and climate.

Some EOs have been used as flavours in food beverages or for food preservation and are considered as safe for consumption at the concentrations used.

WO00/03606 discloses the use of compositions comprising EOs as breathfresheners in pet food. This effect is based on the sweet odour of the EOs described. WO00/03606 also discloses (in Example 2) that eucalyptus oil has a significant detrimental effect of growth of the bacteria Porphyromanos canoris, Veillonella alcalescens, Bacteriodes oralis and Fusobacterium nucleatum. However, the document does not disclose or suggest that essential oils have a detrimental effect on growth of GHLOs, nor does it indicate that essential oils could be used to combat infection by GHLOs.

DE19716660 discloses an herbal preparation for treating *Helicobacter pylori* infections containing essential oil(s) and/or plant extract(s), e.g. eucalyptus oil, useful for treating gastro-intestinal ulcers and gastritis. This document does not disclose or suggest that the particular essential oils according to embodiments of the present invention could have a remarkably superior effect compared to other essential oils. In contrast, it simply discloses a plant-based preparation for growth inhibition and/or killing of *Helicobacter pylori* bacteria which contains at least one of the following essential oils and extracts as active agent(s): essential oils of eucalyptus, anise and fennel and extracts of ginger root, St. John's wort, Curcuma longa rhizome, wormwood, lesser centaury, marsh mallow root, artichoke leaves, galingale rhizome, Haronga madagascarensis, rampion, liquorice, dandelion, Kava pepper (Piper methysticum) and cinammon. There is no disclosure or suggestion which could lead logically or plainly to the specific subject matter of the invention.

Consequently, in a first aspect the present invention provides a nutritional composition which comprises an essential oil selected from the group which consists of carrot seeds, cinnamon bark, clove, cumin, eucalyptus, grapefruit, lemongrass guatemala, manuka oil, origano (vulgaris) sage, savory, tarragon, thyme, a combination of two or more thereof and/or a compound isolated from one of the essential oils wherein the compound is selected from the group which consists of alpha-pinene, beta-pinene, carvacrol, citral, citronellal, estragole, eugenol, eukalyptol, farnesol, geranul acetate, geraniol, ginger oleoresine, isoeugenol, limonene, linalool, menthol, nerol, perilla aldehyde, thymol, trans-2-hexenal or a combination of two or more thereof for use in prevention or treatment of infection by a gastric Helicobacter-like organism.

In a second aspect the invention provides a method of production of a composition according to an embodiment of the invention which comprises the steps of blending the components in the required amounts.

In a third aspect the invention provides use of a composition according to an embodiment of the invention in the manufacture of a functional food product or medicament for the prevention or treatment of infection by a gastric Helicobacter-like organism.

In a forth aspect the invention provides a method of prevention or treatment of infection by a gastric Helicobacter-like organism which comprises consuming or administering functional food product or medicament which comprises an effective amount of a composition according to an embodiment of the invention.

Preferably, an embodiment of the grapefruit essential oil is derived from pink or white grapefruit.

Preferably, an embodiment of the thyme essential oil is derived from thyme (red) or thyme (vulgaris).

Preferably, an embodiment of the composition includes a combination of two or more essential oils and/or two or more of the compounds isolated from an essential oil.

Preferably, an embodiment of the composition includes a carrier, diluent or excipient selected from those known in the art.

Preferably, an embodiment of a composition according to the invention is suitable for consumption by a human. An alternative embodiment is suitable for consumption by a companion animal.

Preferably the essential oil is obtained by steam distillation. Suitable starting materials are for example: those mentioned above including herbs, spices, fruits and tea.

An advantage of the present invention is that it provides a composition wherein the oil has a high solubility and a high activity at acidic pH. This provides the advantage that they can be included in an acidic formula (eg: acidified milk). Furthermore, the oil or compounds derived therefrom are naturally occuring - no synthetic compounds are required.

Another advantage of the present invention is that it provides a blend of different EOs having a synergistic activity (against GHLOs).

Yet another advantage of the present invention is that it provides a method of delivering EOs topically in the stomach via the mouth. This overcomes the need for invasive surgical methods of combating infection.

Additional features and advantages of the present invention are described in, and will be apparent from, the description of the presently preferred embodiments which are set out below with reference to the drawings in which:

**Figure 1** shows MBC determinations of EOs after 1h of incubation (MBC= minimal bacterial concentration: lowest concentration at which bacteria are killed (no growth in subculture)).

**Figure 2** shows MBC determinations of EOs after 24h of incubation.

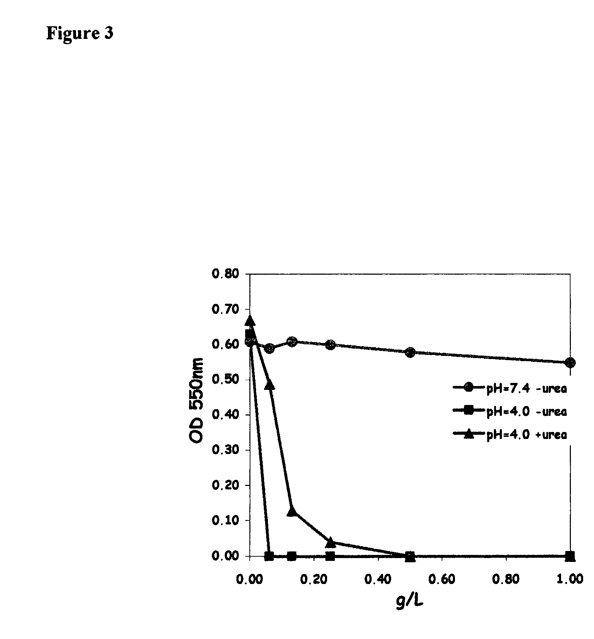

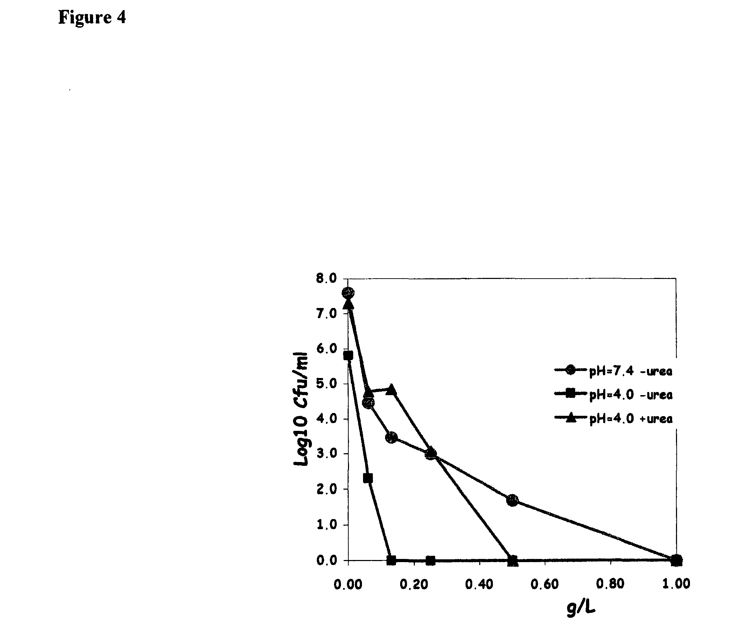

**Figures 3 and 4** show the influence of pH on carrot EO activity. **Figure 3** shows the results of an *H. pylori* urease test and **Figure 4** shows the results of an *H. pylori* viability test. Remarkably, carrot EO is more active at an acidic pH.

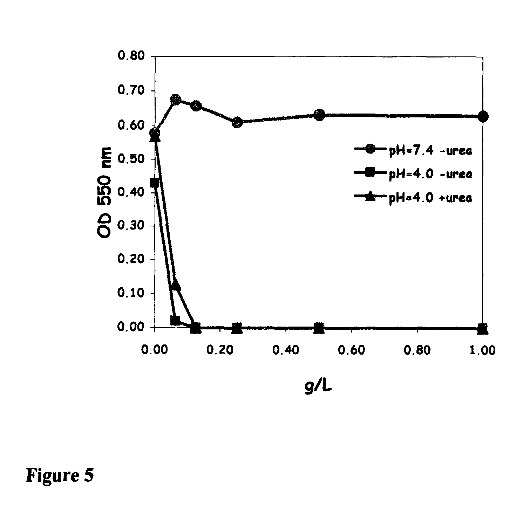

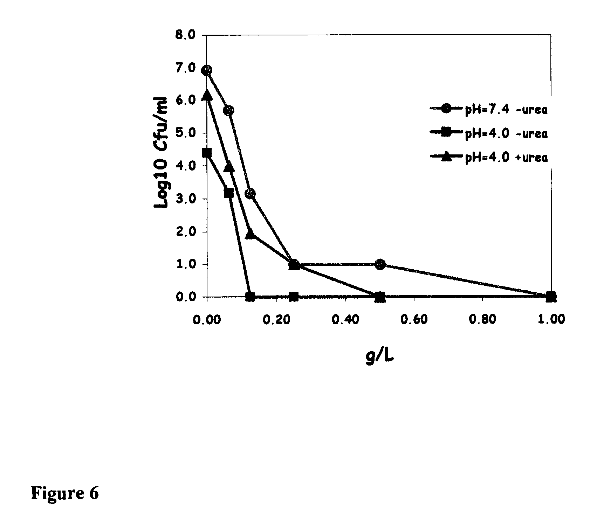

**Figures 5 and 6** show the influence of pH on lemongrass EO activity. **Figure 5** shows the results of an *H. pylori* urease test and **Figure 6** shows the results of an *H. pylori* viability test. Remarkably, lemongrass EO is more active at acidic pH.

Without wishing to be bound by theory it is postulated that the mechanisms of action of EOs could involve alteration of the bacterial membrane integrity (lipophylic compounds) which provide an increased permeability and leakage of intracellular components. In addition, they could involve impairment of enzymatic systems responsible for energy production, synthesis of structural components and/or DNA synthesis. Furthermore, they could be involved in destruction of genetic material.

The parameters used to define the inhibitory capacity of a given substance are: 1) the minimal inhibitory concentration (MIC) which is the lowest concentration at which bacterial growth is prevented, and 2) the minimal bacterial concentration (MBC) which is the lowest concentration at which bacteria are killed (no growth in subculture). In the light of this: MIC ≤ MBC. ?

The following methods of analysis have been used:
a) Diffusion in agar solid medium (disk inhibition assay); and
b) Liquid medium assay (used for MBC determination)

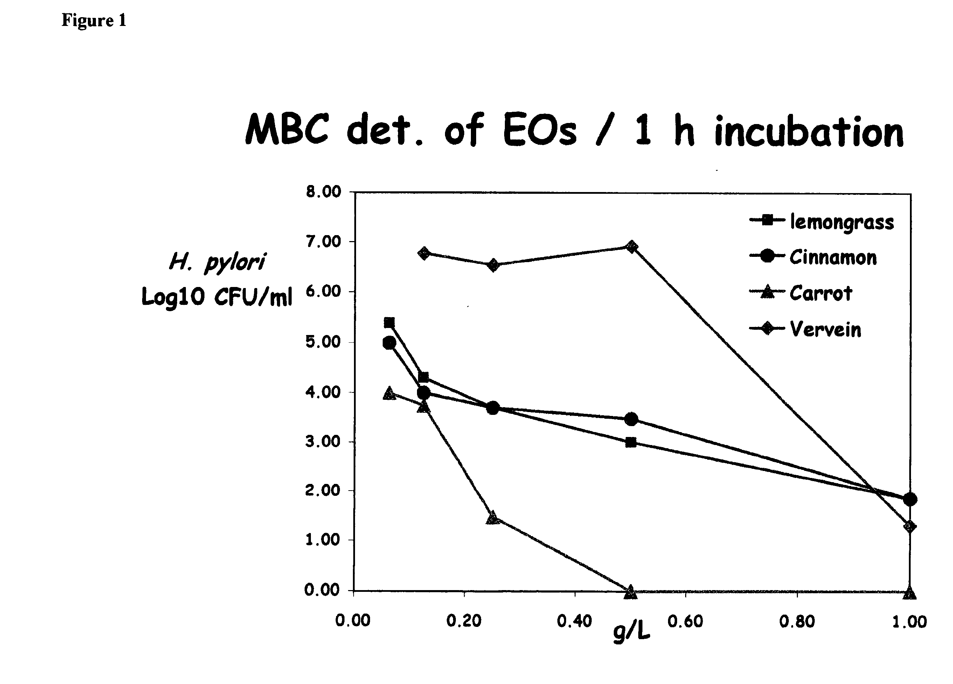

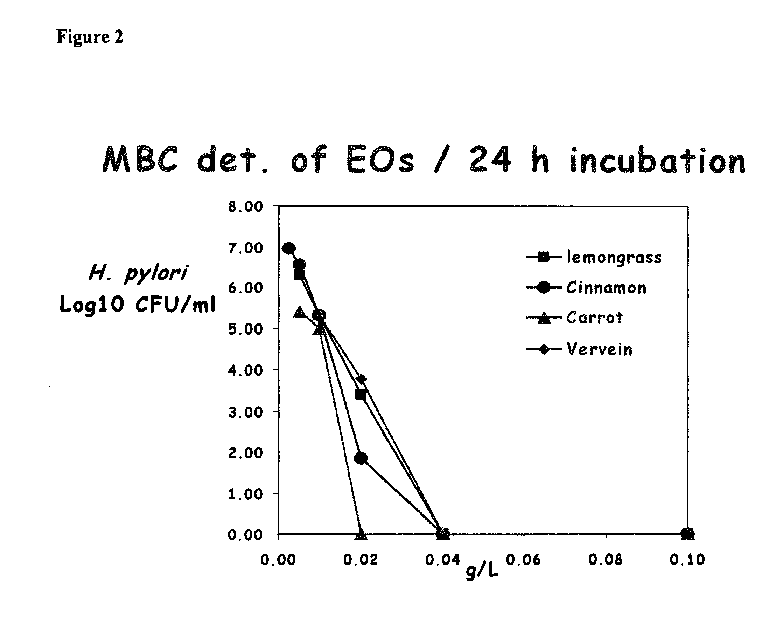

MBC determination in *H. pylori* was carried out by placing bacteria and different dilutions of EO at pH 7.4 together for 1h or 24h, plating (subculture in solid medium) for 3-5 days, and counting colony forming units (CFU). The results were then plotted as *H. pylori* (log10CFU/ml) vs concentration of EO (g/l). It is clear from the plots (shown in **Figures 1 and 2**) that EOs already display a marked effect on *H. pylori* viability after 1h incubation and that the selected EOs are remarkably potent inhibitors *of H. pylori in vitro.*

The following tables show comparative data illustrating that EOs and pure compounds used in accordance with the present invention have a remarkably superior effect compared to the EOs which fall outside the scope of the invention.

**Table 1** illustrates results obtained with EOs and **Table 2** illustrates results obtained with pure compounds found in the EOs. **Table 3** illustrates the results of MBC vs typical concentrations in embodiments of compositions according to the invention. **Table 4** illustrates the results of an MBC (24h) study on *H. pylori* inhibition: EOs vs extracts.

**Table 1**

| | ***Helicobater pylori* inhibition by EOs** | | |
|---|---|---|---|
| | **Diffusion in solid medium (cm)** | | **Liquid medium** |
| **Essential oil** | **1/10 in EtOH** | **1/10 in PG** | **MBC g/L** |
| Carrot seeds | 1.6 | 0.75 | 0.02 |
| Cinnamon bark | 6.3 | 4.5 | 0.04 |
| Clove | not done | 3 | 0.1 |
| Cumin | 0 | 1.2 | 0.1 |
| Eucalyptus | 1.2 | 1 | >0.1 |
| Grapefruit (pink) | difuse | 0.9 | 0.1 |
| Grapefruit (white) | 1.7 | 0.8 | 0.1 |
| Lemongrass Guatemala | 2.9 | 3.2 | 0.04 |
| Manuka oil | not done | 2 | 0.04 |
| Origano (vulgaris) | difuse | 1.5 | 0.04 |
| Sage | 0 | 0.65 | 0.1 |
| Savory | 1.3 | 2.5 | 0.04 |
| Tarragon | 0 | 0.7 | 0.1 |
| Thyme (red) | not done | 1.9 | 0.1 |
| Thyme (vulgaris) | 1.2 | 1.2 | 0.04 |
| **0cm ⇒ ≤ 0.6cm (diameter of disk) = no inhibition.** | | | |

**Table 2**

| | **Liquid medium** | |
|---|---|---|
| **Pure compounds** | **MBC** | **MBC** |
| | **g/L** | **ppm** |
| Alpha-pinene | 0.1 | 100 |
| Beta-pinene | 0.1 | 100 |
| Carvacrol | 0.04 | 40 |
| Citral | 0.04 | 40 |
| Citronellal | 0.1 | 100 |
| Estragole | 0.1 | 100 |
| Eugenol | 0.1 | 100 |
| Eukalyptol | >>0.1 | >>100 |
| Farnesol | 0.04 | 40 |
| Geranyl acetate | >>0.1 | >>100 |
| Geraniol | 0.1 | 100 |
| Ginger oleoresine | ^{≤}0.02 | ^{≤}20 |
| Isoeugenol | 0.04 | 40 |
| Limonene | 0.1 | 100 |
| Linalool | 0.1 | 100 |
| Menthol | >>0.1 | >>100 |
| Nerol | 0.04 | 40 |
| Perilla aldehyde | ^{≤}0.02 | ^{≤}20 |
| Thymol | 0.1 | 100 |
| Trans-2-hexenal | 0.04 | 40 |

**Table 3**

| | | **Usage levels (Fenaroli)** | | | |
|---|---|---|---|---|---|
| **Essential oil** | **MBC (24 h) ppm** | **Drinks ppm** | **Alcoh. drinks ppm** | **Soups ppm** | **Dishes ppm** |
| Corrot seeds | 20 | 4 | 14 | | 22 (meat) |
| Cinnamon bark | 40 | 38.24 | 572.6 | 25 | 288.9 (baked) |
| Clove (leaves) | 100 | 14.50 | 198 | 9.86 | |
| Cumin | 100 | | | | |
| Eukalyptus | > 100 | 2.17 | 2.07 | | 1958 (candies) |
| Grapefruit (pink) | 100 | 276.4 | 132.8 | | 860 (baked) |
| Grapefruit (white) | 100 | 276.4 | 132.8 | | 860 (baked) |
| Lemongrass Guatemala | 40 | 8.99 | 8.94 | | 36.2 (baked) |
| Manuka oil | 40 | | | | |
| Origano (vulgaris) | 40 | | | | |
| Sage | 100 | 10.21 | 5.32 | | 34.24 (baked) |
| Savory | 40 | | | | |
| Tarragon | 100 | 133 | 154 | | 146 (dairy) |
| Thyme (red) | 100 | 4.97 | 5.05 | 2.95 | 2.95 (baked) |
| Thyme (vulgaris) | 40 | 4.98 | 5.02 | | 29.78 (baked) |
| Vervein | 40 | | | | |

**Table 4**

| | **MBC (24 h)** |
|---|---|
| | **g/L** |
| Origan Essential oil | 0.04 |
| Origan-PG extract | 1.0 |
| Origan-MCT extract | > 1.0 |
| Lemongrass Essential oil | 0.04 |
| Lemongrass-PG extract | 1 |
| Lemongrass-MCT extract | > 1.0 |

In an embodiment, a nutritional composition according to an embodiment of the invention comprises a source of protein. Dietary protein is preferred as a source of protein. The dietary protein may be any suitable dietary protein; for example animal protein (such as milk protein, meat protein or egg protein); vegetable protein (such as soy protein, wheat protein, rice protein, and pea protein); a mixture of free amino acids; or a combination thereof. Milk proteins such as casein, whey proteins and soy proteins are particularly preferred.

The composition may also comprise a source of carbohydrates and/or a source of fat.

Preferably, an embodiment of the composition includes a lipid source. The lipid source preferably provides about 5% to about 55% of the energy of the composition; for example about 20% to about 50% of the energy. Lipids making up the lipid source may be any suitable fat or fat mixture. Vegetable fat is particularly suitable; for example soy oil, palm oil, coconut oil, safflower oil, sunflower oil, corn oil, canola oil, lecithins, and the like. Animal fat such as milk fat may also be added if desired.

The lipid source may be any lipid or fat which is suitable for use in a food product. Typical lipid sources include milk fat, safflower oil, egg yolk lipid, canola oil, olive oil, coconut oil, palm oil, palm kernel oil, palm olein, soybean oil, sunflower oil, fish oil, and microbial fermentation oil containing long-chain, polyunsaturated fatty acids. These oils may be in the form of high oleic forms such as high oleic sunflower oil and high oleic safflower oil. The lipid source may also be in the form of fractions derived from these oils such as palm olein, medium chain triglycerides (MCT), and esters of fatty acids such as arachidonic acid, linoleic acid, palmitic acid, stearic acid, docosahexaeonic acid, linolenic acid, oleic acid, lauric acid, capric acid, caprylic acid, caproic acid, and the like.

Preferably, the lipid source comprises medium chain triglycerides; for example in an amount of about 15% to about 35% by weight of the lipid source.

The lipid source preferably has a ratio of n-6 to n-3 fatty acids of about 5:1 to about 15:1; for example about 8:1 to about 10:1.

A source of carbohydrate may be added to the nutritional composition. It preferably provides about 40% to about 80% of the energy of the nutritional composition. Any suitable carbohydrate may be used, for example sucrose, lactose, glucose, fructose, corn syrup solids, maltodextrin, or a mixture thereof.

Dietary fibre may also be added if desired. If added, it preferably comprises up to about 5% of the energy of the nutritional composition. The dietary fibre may be from any suitable origin, including for example soy, pea, oat, pectin, guar gum, gum arabic, fructooligosaccharide or a mixture thereof.

Suitable vitamins and minerals may be included in the nutritional composition in an amount to meet the appropriate guidelines.

One or more food grade emulsifiers may be included in the nutritional composition if desired; for example diacetyl tartaric acid esters of mono- and di-glycerides, lecithin and mono- or di-glycerides or a mixture thereof. Similarly suitable salts and/or stabilisers may be included.

The nutritional composition is preferably enterally administrable; for example in the form of a powder, a liquid concentrate, or a ready-to-drink beverage. If it is desired to produce a powdered nutritional formula, the homogenized mixture is transferred to a suitable drying apparatus such as a spray drier or freeze drier and converted to powder.

A liquid food product may be enriched with the an embodiment of the composition, for example, a drink, a soup, a liquid tea, a fermented milk, a renneted milk, a soy-based product, non-milk fermented product, or a nutritional supplement for clinical nutrition.

Alternatively, a solid food product may be enriched with an embodiment of the composition, for example, a soup, died tea, milk powder, a yogurt, a fresh cheese, a soy-based product, a confectionery bar, a candy, breakfast cereal flakes or bars, or a nutritional supplement for clinical nutrition.

An embodiment of the composition may be included in article of confectionery, for example a sweet or sweetened beverage.

The following examples are given by way of illustration only and in no way should be construed as limiting the subject matter of the present application. Percentages and parts are by weight unless otherwise indicated.

### Example 1: Nutritional Composition.

A composition was made by blending the required ingredients. Its composition is indicated below:

| | |
|---|---|
| Tea based flavored beverage | % |
| Water | 89 |
| HFCS | 10 |
| Powdered tea | 0.2 |
| Tea essence | 0.2 |
| EO mixture | 0.2 (0.01-0.5) |

The EO mixture comprises essential oils from cinnamon bark, clove, pink grapefruit, white grapefruit, lemongrass, vervein, manuka (one EO alone or a combination of two or more).

### Example 2: Nutritional Composition.

A composition was made by blending the required ingredients. Its composition is indicated below:

| | |
|---|---|
| Vegetable soup | % |
| Potato flakes | 50 |
| Dehydrated vegetable | 20 |
| Corn starch | 20 |
| Peanut oil | 9.8 |
| EO mixture | 0.2 (0.01-0.5) |

The EO micture comprises essential oils from carrot seeds, clove, cumin, manuka, oregano, sage, savory, thyme (red and vulgaris), tarragon (one EO alone or a combination of two or more).

### Example 3: Nutritional Composition.

A composition was made by blending the required ingredients. Its composition is indicated below:

| | |
|---|---|
| High boiled candy | % |
| Water | 11 |
| Crystal sugar | 40 |
| Glucose sirop | 40 |
| Fat | 8.6 |
| Emulsifier | 0.2 |
| EO mixture | 0.2 (0.01-0.5) |

The EO micture comprises essential oils from cinnamon bark, clove, pink grapefruit, white grapefruit, lemongrass, vervein, eucalytus (one EO alone or a combination of two or more).

In each example, EO mixture = a combination of the EOs according to an embodiment of the invention in a suitable food grade carrier (solvent, sugar syrup, emulsions). Preferably, at least one pure compound isolated from one of the essential oils is included.

Preferably, the maximal amount of EO is 5000 ppm containing 1-100 % EO(s) / 0-99% carrier

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. A nutritional composition which comprises an essential oil selected from the group which consists of carrot seeds, cinnamon bark, clove, cumin, eucalyptus, grapefruit, lemongrass guatemala, manuka oil, origano (vulgaris) sage, savory, tarragon, thyme, a combination of two or more thereof and/or a compound isolated from one of the essential oils wherein the compound is selected from the group which consists of alpha-pinene, beta-pinene, carvacrol, citral, citronellal, estragole, eugenol, eukalyptol, farnesol, geranul acetate, geraniol, ginger oleoresine, isoeugenol, limonene, linalool, menthol, nerol, perilla aldehyde, thymol, trans-2-hexenal or a combination of two or more thereof for use in prevention or treatment of infection by a gastric Helicobacter-like organism.

2. A nutritional composition according to claim 1 wherein the grapefruit essential oil is derived from pink or white grapefruit.

3. A nutritional composition according to claim 1 wherein the thyme essential oil is derived from thyme (red) or thyme (vulgaris).

4. A nutritional composition according to claim 1 which includes a combination of two or more essential oils and/or two or more of the compounds isolated from an essential oil.

5. A nutritional composition according to any preceding claim which includes a carrier, diluent or excipient.

6. A nutritional composition according to any preceding claim in a form suitable for consumption by a human or companion animal.

7. A method of production of a composition according to an embodiment of the invention which comprises the steps of blending the components in the required amounts.

8. A method according to claim 7 which includes the step of obtaining the essential oil by steam distillation.

9. Use of a composition according to any one of claims 1 to 6 in the manufacture of a functional food product or medicament for the prevention or treatment of infection by a gastric Helicobacter-like organism.

10. A method of prevention or treatment of infection by a gastric Helicobacter-like organism which comprises the step of consuming or administering a functional food product or medicament which comprises an effective amount of a composition according to any one of claims 1 to 6.
